(19) Europäisches Patentamt
European Patent Office
Office européen des brevets

(11) Veröffentlichungsnummer: **0 220 129 B1**

(12) # EUROPÄISCHE PATENTSCHRIFT

(45) Veröffentlichungstag der Patentschrift: **21.08.91**

(51) Int. Cl.$^5$: **C07D 457/12**, A61K 31/48

(21) Anmeldenummer: **86730147.5**

(22) Anmeldetag: **18.09.86**

Die Akte enthält technische Angaben, die nach dem Eingang der Anmeldung eingereicht wurden und die nicht in dieser Patentschrift enthalten sind.

(54) **12- und 13-substituierte Ergolinderivate.**

(30) Priorität: **19.09.85 DE 3533672**

(43) Veröffentlichungstag der Anmeldung:
**29.04.87 Patentblatt 87/18**

(45) Bekanntmachung des Hinweises auf die Patenterteilung:
**21.08.91 Patentblatt 91/34**

(84) Benannte Vertragsstaaten:
**AT BE CH DE FR GB IT LI LU NL SE**

(56) Entgegenhaltungen:
**EP-A- 0 048 695**
**EP-A- 0 056 358**
**EP-A- 0 118 848**
**WO-A-82/02892**

**WEYGAND/HILGETAG: "Organisch-chemische Experimentierkunst", 4. Auflage, Seiten 776-781; (Herausgeber: G. HILGETAG et al.); Johann Ambrosium Barth, 1970, Leipzig; "Lithiumorganische Verbindungen"**

(73) Patentinhaber: **SCHERING AKTIENGESELLSCHAFT Berlin und Bergkamen
Müllerstrasse 170/178 Postfach 65 03 11
W-1000 Berlin 65(DE)**

(72) Erfinder: **Sauer, Gerhard, Dr.
Königsbacher Zeile 41A
W-1000 Berlin 28(DE)**
Erfinder: **Heindl, Josef, Dr.
Eitel-Fritz-Strasse 40
W-1000 Berlin 38(DE)**
Erfinder: **Schröder, Gertrud, Dr.
Theodor-Francke-Strasse 3
W-1000 Berlin 42(DE)**
Erfinder: **Wachtel, Helmut, Dr.
Asternplatz 2
W-1000 Berlin 45(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 12- oder 13-substituierte Ergolinderivate der allgemeinen Formel I, ihre Herstellung und ihre Verwendung als Arzneimittel.

Die erfindungsgemäßen Verbindungen haben die allgemeine Formel I

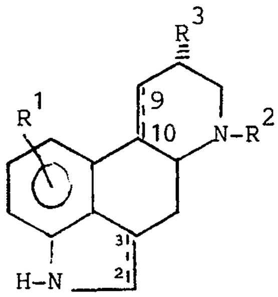

(I)

worin
$R^1$ eine OR'-Gruppe (R' = H, $C_{1-6}$-Alkyl, $C_{2-5}$-Alkanoyl), eine SH, $SR^5$ oder $SOR^5$-Gruppe ($R^5$ = $C_{1-6}$-Alkyl, Phenyl, Phenyl-$C_{1-2}$-Alkyl), eine

$\overset{X}{\overset{\|}{C}}-R^6$-Gruppe,

worin x = O oder S bedeutet und $R^6$ = H, $CF_3$, $C_{1-6}$Alkyl, eine gegebenenfalls mit $C_{1-6}$-Alkyl substituierte Aminogruppe oder $OR^7$ bedeutet mit $R^7$ in der Bedeutung von Wasserstoff oder $C_{1-6}$-Alkyl,
eine -$CR^8R^9R^{10}$-Gruppe
mit $R^8$ = H, OH, O-$C_{2-5}$-Alkanoyl, O-$C_{1-6}$-Alkyl, $C_{1-6}$-Alkyl oder eine gegebenenfalls mit $C_{1-6}$-Alkyl substituierte Aminogruppe,
$R^9$ und $R^{10}$ gleich oder verschieden sind und jeweils H, $C_{1-6}$-Alkyl oder Phenyl bedeuten,
eine $SO_2CF_3$-, $Si(CH_3)_3$ oder CN-Gruppe, ein Cl- oder J-Atom bedeutet und
$R^2$ eine $C_{1-6}$-Alkylgruppe ist und
$R^3$ NH-CO-$NEt_2$ oder NH-CS-$NEt_2$ bedeutet und
$C_9...C_{10}$ und $C_2...C_3$ eine CC-Einfach- oder eine C=C Doppelbindung bedeuten und das Wasserstoffatom in 10-Stellung $\alpha$-ständig ist, wenn $C_9...C_{10}$ eine CC-Einfachbindung ist und das Wasserstoffatom in 3-Stellung $\alpha$- oder $\beta$-ständig ist, wenn $C_2...C_3$ eine CC-Einfachbindung ist, sowie deren Säureadditionssalze.

Unter niederen Alkylresten versteht man solche mit bis zu 6 Kohlenstoffatomen, wobei $C_1$-$C_4$ Alkyle bevorzugt sind wie zum Beispiel Methyl, Ethyl, Isopropyl, n-Propyl, n-Butyl, Isobutyl, tert. Butyl. Die Arylreste können ebenso wie die Aralkylreste gegebenenfalls substituiert sein. z.B. mit niederem Alkyl, niederem Alkoxy oder Halogen.

Als bevorzugte Aralkylreste sind solche mit bis zu 2 C-Atomen im Alkylrest anzusehen wie z.B. der Benzyl- und Phenethylrest. Die Acylreste leiten sich von aliphatischen Carbonsäuren mit bevorzugt 2 - 5 Kohlenstoffatomen ab wie Essigsäure, Propionsäure, Buttersäure, Capronsäure und Trimethylessigsäure.

Die gegebenenfalls mit niederem Alkyl substituierte Aminogruppe kann ein- oder zweifach substituiert sein.

Die Salze der erfindungsgemäßen Verbindungen der Formel I sind Säureadditionssalze und leiten sich von üblicherweise verwendeten Säuren ab. Solche Säuren sind zum Beispiel anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, β-Hydroxybutyrat, Glycolat, Malat, Tartrat, Methansulfonat, Propansulfonat, Naphthalin-1-sulfonat oder Naphthalin-2-sulfonat.

Aus EP-A-118848, EP-A-056358 und WO-A-8202892 sind ähnlich substituierte Ergoline bekannt.

Im Vergleich zu den bekannten in 12- oder 13-Stellung substituierten Ergolinen und dem trans-Dihydro-Lisurid (DE-PS-2238540) besitzen die erfindungsgemäßen Vebindungen der Formel I eine stärkere bzw. mindestens gleich starke zentrale $\alpha_2$-rezeptorblockierende Wirkung bei schwächeren bzw. fehlenden antidopaminergen Effekten. Dieses Wirkprofil läßt die Verbindungen als wertvolle Substanzen zur Behandlung von psychischen Störungen des depressiven Formenkreises erscheinen. Die antidepressive Wirkung der erfindungsgemäßen Verbindungen beruht auf einer zentralen $\alpha$-$_2$Rezeptorblockade, die eine vermehrte Noradrenalinfreisetzung im Gehirn bewirkt und darüber den antidepresssiven Effekt zur Folge hat.

Die zentrale $\alpha_2$-Rezeptorblockade wurde in einem Interaktionstest mit dem $\alpha_2$-Rezeptoragonist Clonidin an Mäusen nach einmaliger i.p. Vorbehandlung dargestellt (Parameter : Aufhebung der durch Clonidin 0,1 mg/kg i.p. verursachten Hypothermie). Männliche NMRI-Mäuse wurden mit verschiedenen Dosen von 1,1-Diethyl-(6-methyl-8α-ergolinyl)-harnstoff (TDHL) bzw. 13-substituierten Ergolinylharnstoffen, die selbst nicht die Thermoregulation der Versuchstiere beeinflussen, bzw. mit Trägermedium vorbehandelt. 30 Minuten später erhielten alle Tiere Clonidin 0,1 mg/kg i.p. 60 Minuten nach Prüfsubstanz bzw. Trägermedium (= 30 Minuten nach Clonidin) wurde die Rektaltemperatur mit Hilfe einer Thermosonde gemessen. Während die mit Trägermedium vorbehandelten Mäuse eine Hypothermie aufwiesen, war an mit TDHL bzw. 13-substituierte- Ergolinylharnstoffen vorbehandelten Tieren der körpertemperatursenkende Effekt des Clonidin dosisabhängig aufgehoben. Wie aus Tabelle 1 ersichtlich, war der clonidinantagonistische Effekt nach 13-$SCH_3$-TDHL in der Dosierung 0,78 mg/kg statistisch signifikant.

Die zentrale Dopaminrezeptorblockade wurde in einem Interaktionstest mit dem Dopaminrezetoragonisten Apomorphin an Mäusen nach einmaliger i.p.-Vorbehandlung dargestellt. Parameter : Aufhebung der durch Apomorphin 5 mg/kg i.p. verursachten Hypothermie). Das weitere Vorgehen glich dem bei der zentralen $\alpha_2$-Rezeptorblockade dargestellten Verfahren.

Wie aus Tabelle 2 ersichtlich, war der apomorphinantagonistische Effekt nach TDHL in der Dosierung 3,13 mg/kg statistisch hochsignifikant. 13-$SCH_3$-TDHL wirkte in Dosierungen 0,1 - 3,13 mg/kg nicht apomorphinantagonistisch.

Aufgrund dieser Befunde können die erfindungsgemäßen Verbindungen daher als Neuroleptika zur Behandlung von Psychosen des schizophrenen Formenkreises oder als Antidepressiva verwendet werden.

Ferner zeigen die erfindungsgemäßen Verbindungen blutdrucksenkende Wirkung und eignen sich daher als Arzneimittel zur Hochdrucktherapie.

In tierpharmakologischen Untersuchungen zeigte beispielsweise 1,1-Diethyl-(13-hydroxy-6-methyl-8α-ergolinyl)-harnstoff (13-OH-TDHL) eine dosisabhängige Blutdrucksenkung an spontan-hypertensiven Ratten, die nach einer Methode modifiziert nach Weeks präpariert wurden (Weeks, J.R., Routine Direct Measurement of Arterial Pressure in Anaesthetized Rats. Proc.S.Exp.Biol.Med. 104:646-648, 1960).

Zur Untersuchung der blutdrucksenkenden Wirkung und ihrer Dosisabhängigkeit wurden folgende pharmakologische Tests ausgeführt:

Bei ca. 300 g schweren, männlichen SH-Ratten wurde mittels eines implantierten Aortenkatheters der mittlere arterielle Blutdruck und die Herzfrequenz ermittelt.

Die Prüfsubstanz wurde über einen in der Vena Jugularis plazierten Katheter im Bolus intravenös in den Dosierungen 0,01 mg/kg, 0,1 mg/kg und 1,0 mg/kg KM appliziert, nachdem sie in DMSO gelöst und auf das zu verabreichende Volumen mit Aqua dest. aufgefüllt war.

Die maximale Blutdrucksenkung nach Verabreichung von 1,0 mg/kg KM beträgt am Beispiel des 13 OH-TDHL 35 % des Ausgangswertes; bei dieser Dosis hält die blutdrucksenkende Wirkung bis zum Ende des Versuches nach 120 Minuten an. Die Herzfrequenz wird in der Dosis von 1 mg/kg KM um maximal 30 % gesenkt.

Tabelle 1

Antagonistische Wirkung der Vorbehandlung (30 min. i.p.) mit verschiedenen Dosen von 13-substituierten Ergolinyl-harnstoffen auf die durch Clonidin (0.1 mg/kg i.p.) ausgelösten Hypothermie an Mäusen. Die Rektaltemperatur der Versuchstiere wurde 30 min. nach Clonidin (= 60 min. nach Prüfsubstanz) gemessen (x : p <0.05, xx : p <0.01 vs Kontrolle; Varianzanalyse/Dunnett-Test)

| Substanz | n | Kontrolle | Rektaltemperatur [°C](Mittelwert ± S.E.M.) Prüfsubstanzdosis [mg/kg] 0.05 | 0.1 | 0.2 | 0.39 | 0.78 | 1.56 | 3.13 |
|---|---|---|---|---|---|---|---|---|---|
| TDHL | 8 | 33.1 ± 0.2 | - | - | - | 33.6 ± 0.2 | 33.7 ± 0.3 | 34.1 ± 0.2xx | 34.7 ± 0.3xx |
| $13\text{-}SCH_3\text{-}TDHL$ | 8 | 33.5 ± 0.2 | - | 33.9 ± 0.2 | 34.0 ± 0.3 | 34.4 ± 0.2x | 35.1 ± 0.3xx | 35.5 ± 0.2xx | 35.6 ± 0.3xx |

Tabelle 2 :

Antagonistische Wirkung der Vorbehandlung ( 30 min. i.p.) mit verschiedenen Dosen von 13-substituierten Ergolinharnstoffen auf die dadurch Apomorphin ( 5 mg/kg i.p. ) ausgelöste Hypothermie an Mäusen. Die Rektaltemperatur der Versuchstiere wurde 30 min. nach Apomorphin ( = 60 min. nach Prüfsubstanz ) gemessen (x : $p < 0.05$, xx : $p < 0.01$ vs Kontrolle; Varianzanalyse/Dunnett-Test).

| Substanz | | Rektaltemperatur [°C](Mittelwert ± S.E.M.) Prüfsubstanzdosis [mg/kg] | | | | | | | | |
|---|---|---|---|---|---|---|---|---|---|---|
| | n | Kontrolle | 0.05 | 0.1 | 0.2 | 0.39 | 0.78 | 1.56 | 3.13 | 6.25 |
| TDHL | 8 | 32.5 ± 0.4 | - | - | - | - | 33.9 ± 0.5 | 33.8 ± 0.4 | 35.1 ± 0.5xx | 35.5 ± 0 |
| 13-$SCH_3$-TDHL | 8 | 32.3 ± 0.3 | - | 32.5 ± 0.3 | 31.5 ± 0.1 | 31.9 ± 0.5 | 32.7 ± 0.6 | 33.3 ± 0.4 | 32.8 ± 0.5 | - |

Die Herstellung der Verbindungen der allgemeinen Formel I erfolgt nach an sich bekannten Verfahren, indem man ein 12- oder 13-Br-Ergolinderivat der allgemeinen Formel II

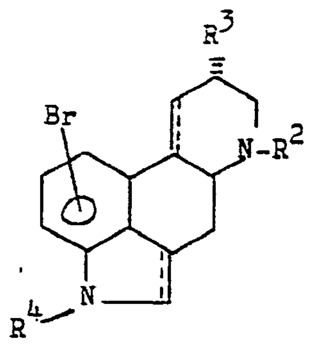

(II)

worin $R^2$, $R^3$ und C---C die obige Bedeutung haben und $R^4$ Wasserstoff oder eine Schutzgruppe ist,mit einem Lithium-Organyl umsetzt und das so erhaltene 12- oder 13-Li-Ergolinderivat der allgemeinen Formel III

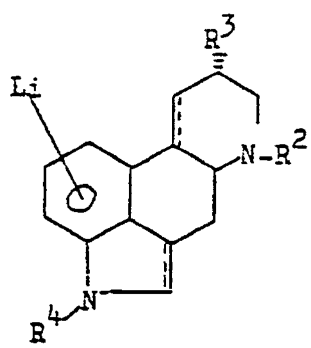

(III)

worin $R^4$, $R^2$, $R^3$ und C---C die obige Bedeutung haben, mit einem elektrophilen Reagenz umsetzt und gewünschtenfalls anschließend eine OH-Gruppe alkyliert oder acyliert, eine Carboxylfunktion verestert, eine Aldehydfunktion reduziert, ein Carbonsäureamid in ein Nitril überführt, ein Schwefelatom oxidiert und/oder einen Harnstoff in einen Thioharnstoff umwandelt und gegebenenfalls mit einer Säure in das physiologisch verträgliche Säureadditionssalz überführt.

Zur Herstellung der Verbindungen der allgemeinen Formel III können alle bekannten Lithium-Organyle verwendet werden, wobei Lithiumalkyle und Lithiumphenyl bevorzugt sind. Als Lithiumalkyl kommt insbesondere tert.-Butyllithium in Frage, wobei man 1 bis 10 Äquivalente verwendet.

Die Reaktion wird in einem aprotischen Lösungsmittel wie Ether oder Kohlenwasserstoff durchgeführt, beispielsweise in Tetrahydrofuran, Dioxan, Diethylether, Toluol, Hexan u.a.

Bewährt hat sich der Zusatz einer stöchiometrischen Menge von Tetramethylethylendiamin bezogen auf Lithiumalkyl.

$R^4$ kann eine üblicherweise verwendete Schutzgruppe darstellen wie z.B. einen Acyl- oder Silylrest, wobei der tri-Alkylsilylrest, insbesondere die tert. Butyldimethylsilylgruppe bevorzugt ist.

Der Brom-Lithium-Austausch wird bei Temperaturen von 20 °C bis - 110 °C vorgenommen, wobei in Gegenwart einer Schutzgruppe Temperaturen von - 70 °C bis - 110 °C und ohne Schutzgruppe 20 °C bis - 70 °C bevorzugt sind.

Zur Vermeidung von Nebenreaktionen kann das Proton am Harnstoff vor dem Brom-Lithium-Austausch mit den üblichen Methoden, wie Z.B. Zusatz von Lithiumdiisopropylamid oder Lithium-bis(trimethylsilyl)-amid in stöchiometrischen Mengen, entfernt werden.

Die Reaktion ist nach ca. 5 Minuten bis 2 Stunden beendet und wird zweckmäßigerweise unter Inertgas wie z.B. Argon oder Stickstoff durchgeführt.

Das so erhaltene Lithium-Ergolinderivat der allgemeinen Formel III wird ohne weitere Aufarbeitung mit dem elektrophilen Reagenz in dem aprotischen Lösungsmittel umgesetzt.

Als elektrophiles Reagenz ist z.B. geeignet:

Thiosulfonsäure-S-ester wie z.B. Methan-Thiosulfonsäure-S-methylester. Toluothiosulfonsäure-S-ethylester, Toluolthiosulfonsäure-S-n-propylester;

Disulfide wie z.B. Dibenzyldisulfid, Diphenyldisulfid. Tetraisopropylthiuramdisulfid;

Isocyanate und Isothiocyanate wie z.B. Trimethylsilylisocyanat, Methylisothiocyanat;
Formamide wie z.B. Dimethylformamid;
Aldehyde wie z.B. Benzaldehyd;
Immoniumverbindungen wie z.B. N,N-Dimethyl-methylen-immonium-jodid, N,N-Dimethyl-methylen-immonium-chorid;
Halogenide wie z.B. Alkylhalogenide beispielsweise Methyljodid, Isopropyljodid, Alkylbromid. halogenierte Silane beispielsweise Trimethylchlorsilan, Säurechloride beispielsweise Acetylchlorid, Halogenierungsmittel beispielsweise N-Chlor-Succinimid, N-Jodsuccinimid und Halogen beispielsweise Jod;
Anhydride wie z.B. Trifluoressigsäureanhydrid, Trifluormethansulfonsäureanhydrid;
Borsäuretrimethylester;
Nitrobenzol;
$CO_2$ u.a.

Stellt der Reaktant unter normalen Bedingungen ein Gas dar, so wird dieses gasförmig eingeleitet oder in fester Form wie z.B. als festes Kohlendioxid zur Reaktion gebracht.

Die elektrophile Substitution wird bei tiefen Temperaturen (0 °C bis - 90 °C) durchgeführt und anschließend wird gegebenenfalls bei Raumtemperatur ca. 2 Stunden nachgerührt.

Falls vorhanden, kann die Schutzgruppe $R^4$ nach den üblichen Methoden durch Behandeln mit Säuren, wie verdünnter Mineralsäure, Trifluoressigsäure, oder anorganischen Basen, wie KOH, NaOH, oder Fluorid wie Tetrabutylammoniumfluorid in inerten Lösungsmitteln, z.B. Wasser, Alkoholen, Kohlenwasserstoffen, u.a. bei Raumtemperatur abgespalten werden.

Wird Borsäuretrialkylester als elektrophiles Reagenz eingesetzt, so muß zur Einführung der OH-Gruppe eine Lösung von $H_2O_2$ zugesetzt werden.

Gewünschtenfalls können anschließend die Substituenten in 12-und in 13-Stellung nach an sich bekannten Methoden umgesetzt werden.

Beispielsweise können die OH-Gruppen alkyliert werden z.B. mit einem Alkylhalogenid in Lösungsmitteln wie DMF, DMSO, Aceton, in Gegenwart von Basen wie NaH, $K_2CO_3$ bei Raumtemperatur oder erhöhter Temperatur oder acyliert werden z.B. mit Säurechloriden oder Säureanhydriden in Gegenwart von Aminen oder Pyridin bei Raumtemperatur.

Carbonsäureamide können in Nitrile überführt werden z.B. durch Umsetzung mit Phosphoroxychlorid ohne Lösungsmittel oder in einem aprotischen Lösungsmittel wie Ether, Methylenchlorid bei Raumtemperatur oder erhöhter Temperatur.

Bedeutet $R^1$ eine Alkylmercaptangruppe, so kann diese zur Sulfinylgruppe oxidiert werden, indem man z.B. mit Na-metaperjodat in einem inerten Lösungsmittel wie Acetonitril, Dioxan, THF bei Raumtemperatur oder erhöhter Temperatur zum Sulfoxid oxidiert.

Bedeutet $R^1$ eine Aldehydgruppe, so kann diese zum entsprechenden Alkohol reduziert werden, indem man z.B. mit Lithiumaluminiumhydrid in einem aprotischen Lösungsmittel wie Ether beispielsweise THF, Diethylether bei Raumtemperatur die Reduktion durchführt.

Bedeutet $R^1$ eine Carbonsäuregruppe, so kann man diese durch Lösen in einem Alkohol, wie z.B. Methanol oder Ethanol in Anwesenheit einer Säure, wie Chlorwasserstoff oder p-Toluolsulfonsäure verestern.

Die Überführung der 8α-Harnstoffderivate in die entsprechenden Thione erfolgt durch Umsetzung mit Phosphoroxychlorid und nachfolgender Reaktion mit Kaliumxanthogenat. Die Reaktion wird bei tiefen Temperaturen mit zwischenzeitlicher Temperaturerhöhung in inerten Lösungsmitteln wie Ethern vorgenommen.

Zur Bildung von Salzen werden die Verbindungen der Formel I in wenig Methanol oder Methylenchlorid gelöst und mit einer konzentrierten Lösung der gewünschten Säure in Methanol bei Raumtemperatur versetzt.

Zur Verwendung der erfindungsgemäßen Verbindungen als Arzneimittel werden diese in die Form eines pharmazeutischen Präparats gebracht, das neben dem Wirkstoff ein für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs. Stabilisierungs-, Netzmittel oder Emulgatoren, Salze zur Veränderung des osmotischen Drucks oder Puffer.

Die Herstellung der Ausgangsverbindungen ist bekannt oder erfolgt nach bekannten Methoden.

Die nachfolgenden Beispiele sollen das erfindungsgemäße Verfahren erläutern.

Herstellung der Ausgangsverbindungen

3-(13-Brom-1- tert.-butyl-dimethylsilyl -6-methyl-8α-ergolinyl)-1,1-diethyl-harnstoff

Man stellt sich eine Lösung von Lithiumdiisopropylamid aus 400 ml frisch destilliertem, wasserfreien THF, 12,9 ml wasserfreiem Diisopropylamin und 42,8 ml n-Butyllithium (in Hexan) bei 0 °C her. Diese Lösung wird auf - 20 °C gekühlt, mit 8,21 g 3-(13-Brom-6-methyl-8α-ergolinyl)-1,1-diethyl-harnstoff (19,5 mMol), gelöst in 200 ml frisch destilliertem, wasserfreien THF, bei - 20 °C versetzt und 15 Minuten gerührt. Bei der gleichen Temperatur gibt man die Lösung von 10,7 g tert.-Butyldimethylsilylchlorid in 150 ml frisch destilliertem wasserfreien THF zu und rührt wieder 15 Minuten. Dann wird der Ansatz auf Eis gegossen, mit 25 %igem Ammoniak alkalisch gemacht und mit Methylenchlorid ausgeschüttelt. Man chromatographiert an Kieselgel mit Hexan, Diisopropylether, Methylenchlorid und Methanol und erhält nach Kristallisation aus Essigester und Diisopropylether 7,3 g (70 % d. Th.).
$[\alpha]_D$ = - 12 ° (0,5 % in Chloroform)

In analoger Weise werden die folgenden Silylverbindungen dargestellt:
3-(13-Brom-1- tert.-butyl-dimethyl-silyl -6-n-propyl-8α-ergolinyl)-1,1-diethyl-harnstoff
3-(12-Brom-1- tert.-butyldimethylsilyl -6-methyl-8α-ergolinyl)-1,1-diethyl-harnstoff
$[\alpha]_D$ = + 34 ° (0,5 % in Chloroform)
3-(12-Brom-1- tert.-butyl-dimethyl-silyl -9,10-didehydro-6-methyl-8α-ergolinyl)-1,1-diethyl-harnstoff
3-(13-Brom-1- tert.-butyl-dimethyl-silyl -2,3-dihydro-6-methyl-8α-ergolinyl)-1,1-diethyl-harnstoff
3-(12-Brom-1- tert.-butyl-dimethyl-silyl -2,3-dihydro-6-methyl-8α-ergolinyl)-1,1-diethyl-harnstoff

Beispiel 1

1,1-Diethyl-3-(6-methyl-13-methylthio-8α-ergolinyl)-harnstoff

3,2 ml (15 mMol) destilliertes Hexamethyldisilazan wird in 40 ml wasserfreiem, frisch destillierten Toluol unter Argon vorgelegt. Nach dem Abkühlen dieser Mischung auf 0 °C tropft man 8,5 ml (14 mMol) 15 %iges n-Butyllithium in Hexan dazu und rührt noch 15 Minuten bei 0 °C. Anschließend tropft man die Lösung von 5,33 g 3-(13-Brom-1- tert.-butyl-dimethyl-silyl -6-methyl-8α-ergolinyl)-1,1-diethyl-harnstoff (10 mMol) in 200 ml wasserfreiem, frisch destillierten Toluol zu und rührt 15 Minuten bei 0 °C nach. Dann gibt man 10 ml destilliertes Tetramethylethylendiamin zu und kühlt auf - 90 °C ab. Jetzt werden 50 ml 1,4 m tert.-Butyllithium ( 70 mMol) zugegeben und 2 Minuten gerührt.

Zur Lösung des 13-Lithium-ergolinyl-harnstoffes gibt man die Lösung von 6,3 g Methanthiosulfonsäure-S-methylester (10 mMol) in 50 ml frisch destilliertem, wasserfreien THF. Nach 10 Minuten Rühren gibt man die Mischung auf Eis, macht mit 25 %iger Ammoniaklösung alkalisch und schüttelt mit Methylenchlorid aus.

Das Rohprodukt wird zur Entsilylierung in 500 ml Methanol gelöst und mit 250 ml 7n Kalilauge 15 Minuten bei Raumtemperatur gerührt. Wieder wird der Ansatz auf Eis gegeben und mit Methylenchlorid ausgeschüttelt. Nach Abdestillieren des Lösungsmittels wird der Rückstand chromatographiert (Ausbeute 1,27 g, 33 % d. Th.) und aus Essigester und Diisopropylether kristallisiert. Ausbeute 0,7 g (18% d.Th.)
$[\alpha]_D$ = - 13 ° (0,25 % in Chloroform).

In völlig analoger Weise werden aus den folgenden 12- und 13-Bromergolinyl-harnstoffen mit Methanthiosulfonsäure-S-methylester dargestellt:

Aus 3-(12-(Brom-1- tert.-butyl-dimethyl-silyl -6-methyl-8α-ergolinyl)-1,1-diethyl-harnstoff 1,1-Diethyl-3-(6-methyl-12-methylthio-8α-ergolinyl)-harnstoff, Ausbeute 44 % d.Th., $[\alpha]_D$ = + 24 ° (0,5 % in Chloroform).

Aus 3-(12-Brom-1- tert.-butyl-dimethyl-silyl -2,3-dihydro-6-methyl-8α-ergolinyl)-1,1-diethyl-harnstoff 1,1-Diethyl-3-(2,3-dihydro-6-methyl-12-methylthio-8α-ergolinyl)-harnstoff, Ausbeute 80 % d.T., $[\alpha]_D$ = + 41,7 ° (0,5 % in Chloroform).

Aus 3-(13-Brom-1- tert.-butyl-dimethyl-silyl -2,3-dihydro-6-methyl-8α-ergolinyl)-1,1-diethyl-harnstoff 1,1-Diethyl-3-(2,3-dihydro-6-methyl-13-methylthio-8α-ergolinyl)-harnstoff

Aus 3-(12-Brom-1- tert.-butyl-dimethyl-silyl -9,10-didehydro-6-methyl-8α-ergolinyl)-1,1-diethyl-harnstoff 3-(9,10-Didehydro-6-methyl-12-methylthio-8α-ergolinyl)-1,1-diethyl-harnstoff

Aus 3-(13-Brom-1- tert.-butyl-dimethyl-silyl -6-n-propyl-8α-ergolinyl)-1,1-diethyl-harnstoff den 1,1-Diethyl-3-(13-methyl-thio-6--n-propyl-8α-ergolinyl)-harnstoff

Aus 3-(13-Brom-1- tert.-butyl-dimethyl-silyl -2,3-dihydro-6-n-propyl-8α-ergolinyl)-1,1-diethyl-harnstoff den 1,1-Diethyl-3-(2,3-dihydro-13-methylthio-6-n-propyl-8α-ergolinyl)-harnstoff

Ersetzt man Methanthiosulfonsäure-S-methylester durch andere Elektrophile, erhält man analog die folgenden Verbindungen:

Mit Toluolthiosulfonsäure-S-ethylester und 3-(12-Brom-1-tert.-butyl-dimethyl-silyl -6-methyl-8α-ergolinyl)-1,1-diethyl-harnstoff den 1,1-Diethyl-3-(12-ethylthio-6-methyl-8α-ergolinyl)-harnstoff, Ausbeute 48 % d.Th., $[\alpha]_D$ = + 98 ° (0,5 % in Chloroform).

Mit Toluothiosulfonsäure-S-n-propylester und 3-(13-Brom-1-tert.-butyl-dimethyl-silyl -6-methyl-8α-ergolinyl)-1,1-diethyl-harnstoff den 1,1-Diethyl-3-(6-methyl-13-n-propylthio-8α-ergolinyl)-harnstoff

Mit Dibenzyldisulfid und 3-(13-Brom-1- tert.-butyl-dimethyl-silyl -6-methyl-8α-ergolinyl)1,1-diethyl-harnstoff den 3-(13-Benzylthio-6-methyl-8α-ergolinyl)1,1-diethyl-harnstoff

Mit Diphenyldisulfid und 3-(13-Brom-1- tert.-butyl-dimethyl-silyl -6-methyl-8α-ergolinyl)-1,1-diethyl-harnstoff den 1,1-Diethyl-3-(6-methyl-13-phenylthio-8α-ergolinyl)-harnstoff

Mit Tetraisopropylthiuramidisulfid und 3-(13-Brom-1- tert.-butyl-dimethyl-sily -6- methyl-8α-ergolinyl)-1,1-diethyl-harnstoff und Hydrolyse mit 20 %iger ethanolischer Kalilauge den 1,1-Diethyl-3-(13-merkapto-6-methyl-8α-ergolinyl)-harnstoff

Mit Nitrobenzol und 3-(13-Brom-1- tert.-butyl-dimethyl-silyl -6-methyl-8α-ergolinyl)1,1-diethyl-harnstoff den 1,1-Diethyl-3-(13-hydroxy-6-methyl-8α-ergolinyl)-harnstoff
Ausbeute 34 % d. Th.,$[\alpha]_D$ = +11 ° (0,5 % in Methanol)

Mit Borsäuretrimethylester und 3-(12-Brom-1- tert.-butyl-dimethyl-silyl -2,3-dihydro-6-methyl-8α-ergolinyl)-1,1-diethyl-harnstoffund anschließende Behandlung mit Wasserstoffperoxid den 1,1-Diethyl-3-(2,3-dihydro-12-hydroxy-6-methyl-8α-ergolinyl)-harnstoff

Mit Nitrobenzol und 3-(13-Brom-1- tert.-butyl-dimethyl-silyl-2,3-dihydro-6-methyl-8α-ergolinyl)-1,1-diethyl-harnstoff den 1,1-Diethyl-3-(2.3-dihydro-13-hydroxy-6-methyl-8α-ergolinyl)-harnstoff

Mit Nitrobenzol und 3-(12-Brom-1- tert.-butyl-dimethyl-silyl-9.10-didehydro-6-methyl-8α-ergolinyl)-1,1-diethyl-harnstoff den 3-(9,10-Didehydro-12-hydroxy-6-methyl-8α-ergolinyl)-1,1-diethyl-harnstoff
Ausbeute 31 % d. Th.

Mit Nitrobenzol und 3-(13-Brom-1- tert.-butyl-dimethyl-silyl-6-n-propyl-8α-ergolinyl)-1,1-diethyl-harnstoff den 1,1-Diethyl-3-(13-hydroxy-6-n-propyl-8α-ergolinyl)-harnstoff

Mit Trimethylsilylisocyanat und 3-(12-Brom-1- tert.-butyl-dimethyl-silyl -6-methyl-8α-ergolinyl)-1,1-diethyl-harnstoff das 8α-(3,3-Diethylureido)-6-methyl-ergolin-12-carbonsäureamid, Ausbeute 15 % d.Th.

Mit Methylisothiocyanat und 3-(13-Brom-1-tert.-butyl-dimethyl-silyl -6-methyl-8α-ergolinyl)-1,1-diethyl-harnstoff des 8α-(3,3-Diethyl-ureido)-6-methyl-ergolin-13-thiocarbonsäure-methylamid
Ausbeute 29 % d. Th. $[\alpha]_D$ = + 32 ° (0,5 % in Chloroform).

Mit Methylisothiocyanat und 3-(13-Brom-1- tert.-butyl-dimethyl-silyl -2,3-dihydro-6-methyl-8α-ergolinyl)-1,1-diethyl-harnstoffdas 8α(3,3-Diethylureido)-2,3-dihydro-6-methyl-ergolin-13-thiocarbonsäure-methylamid

Mit Kohlendioxid und 3-(13-Brom-1- tert.-butyl-dimethyl-silyl-6-methyl-8α-ergolinyl)-1,1-diethyl-harnstoff die 8α-(3,3-Diethylureido)-6-methyl-ergolin-13-carbonsäure

Mit Chlorameisensäuremethylester und 3-(13-Brom-1- tert.-butyl-dimethyl-silyl -6-methyl-8α-ergolinyl)-1,1-diethyl-harnstoff den 8α-(3,3-Diethylureido)-6-methyl-ergolin-13-carbonsäure-methylester

Mit Dimethylformamid und 3-(13-Brom-1- tert.-butyl-dimethyl-silyl -6-methyl-8α-ergolinyl)-1,1-diethyl-harnstoff den 1,1-Diethyl-3-(13-formyl-6-methyl-8α-ergolinyl)-harnstoff
Ausbeute 13 %, $[\alpha]_D$ = - 11° (0,5 % in Chloroform)

Mit Benzaldehyde und 3-(13-Brom-1- tert.-butyl-dimethyl-silyl 6-methyl-8α-ergolinyl)-1,1-diethyl-harnstoff den 1,1-Diethyl-3-[6-methyl-13-(phenyl-hydroxy-methyl)-8α-ergolinyl]-harnstoff

Mit N,N-Dimethyl-methylenimmonium-iodid und 3-(13-Brom-1- tert.-butyl-dimethyl-silyl -6-methyl-8α-ergolinyl)-1,1-diethyl-harnstoff den 1,1-Diethyl-3-(13-dimethyl-aminomethyl-6-methyl-8α-ergolinyl)-harnstoff

Mit N,N-Dimethyl-methylenimmoinium-chlorid und 3-(12-Brom-1-tert.-butyl-dimethyl-silyl -9,10-didehydro-6-methyl-8α-ergolinyl)-1,1-diethyl-harnstoff den 3-(9,10-Didehydro-12-dimethyl-aminomethyl-6-methyl-8α-ergolinyl)-1,1-diethyl-harnstoff

Mit Methyliodid und 3-(12-Brom-1- tert.-butyl-dimethyl-silyl-6-methyl-8α-ergolinyl)-1,1-diethyl-harnstoff den 1,1-Diethyl-3-(6,12-dimethyl-8α-ergolinyl)-harnstoff, Ausbeute 31 %, $[\alpha]_D$ = + 5,5 ° (0,5 % in Chloroform).

Mit Methyliodid und 3-(13-Brom-1- tert.-butyl-dimethyl-silyl-6-methyl-8α-ergolinyl)-1,1-diethyl-harnstoff den 1,1-Diethyl-3-(6,13-dimethyl-8α-ergolinyl)-harnstoff

Mit Isopropyliodid und 3-(13-Brom-1- tert.-butyl-dimethyl-silyl -2,3-dihydro-6-methyl-8α-ergolinyl)-1,1-diethyl-harnstoff den 1,1-Diethyl-3-(6-methyl-13-iso-propyl-8α-ergolinyl)-harnstoff

Mit Isopropyliodid und 3-(13-Brom-1- tert.-butyl-dimethyl-silyl -6-n-propyl-8α-ergolinyl)-1,1-diethyl-harnstoff den 1,1-Diethyl-3-(13-iso-propyl-6-n-propyl-8α-ergolinyl)-harnstoff

Mit Trifluoressigsäureanhydrid und 3-(13-Brom-1- tert.-butyl-dimethyl-silyl -6-methyl-8α-ergolinyl)-1,1-diethyl-harnstoff den 1,1-Diethyl-3-(6-methyl-13-trifluoracetyl-8α-ergolinyl)-harnstoff

Mit Trifluormethansulfonsäureanhydrid und 3-(13-Brom-1- tert.-butyl-dimethyl-silyl -6-methyl-8α-ergolinyl)-1,1-diethyl-harnstoff das [8α-(3,3-Diethylureido)-6-methyl-ergolin-13-yl]-(trifluor-methyl)-sulfon

Mit Trimethylchlorsilan und 3-(13-Brom-1- tert.-butyl-dimethyl-silyl -6-methyl-8α-ergolinyl)-1,1-diethyl-harnstoff den 1,1-Diethyl-3-(6-methyl-13- trimethyl-silyl -8α-ergolinyl)-harnstoff

Mit Acetylchlorid und 3-(13-Brom-1- tert.-butyl-dimethyl-silyl -6-methyl-8α-ergolinyl)-1,1-diethyl-harnstoff den 3-(13-Acetyl-6-methyl-8α-ergolinyl)-1,1-diethyl-harnstoff

Mit N-Chlorsuccinimid und 3-(12-Brom-1- tert.-butyl-dimethyl-silyl -6-methyl-8α-ergolinyl)-1,1-diethyl-harnstoff den 3-(12-Chlor-6-methyl-8α-ergolinyl)-1,1-diethyl-harnstoff

Mit N-Chlorsuccinimid und 3-(13-Brom-1- tert.-butyl-dimethyl-silyl -6-methyl-8α-ergolinyl)-1,1-diethyl-harnstoff den 3-(13-Chlor-6-methyl-8α-ergolinyl)-1,1-diethyl-harnstoff

Mit N-Chlorsuccinimid und 3-(13-Brom-1- tert.-butyl-dimethyl-silyl -2,3-dihydro-6-methyl-8α-ergolinyl)-1,1-diethyl-harnstoffden 3-(13-Chlor-2,3-dihydro-6-methyl-8α-ergolinyl)-1,1-diethyl-harnstoff

Mit N-Chlorsuccinimid und 3-(12-Brom-1- tert.-butyl-dimethyl-silyl -9,10-didehydro-6-methyl-8α-ergolinyl)-1,1-diethyl-harnstoff den 3-(12-Chlor-9,10-didehydro-6-methyl-8α-ergolinyl)-1,1-diethyl-harnstoff

Mit N-Iodsuccinimid und 3-(12-Brom-1- tert.-butyl-dimethyl-silyl -6-methyl-8α-ergolinyl)-1,1-diethyl-harnstoff den 1,1-Diethyl-3-(12-iod-6-methyl-8α-ergolinyl)-harnstoff

Mit Jod und 3-(13-Brom-1- tert.-butyl-dimethyl-silyl -6-methyl-8α-ergolinyl)-1,1-diethyl-harnstoff den 1,1-Diethyl-3-(13-iod-6-methyl-8α-ergolinyl)-harnstoff

Mit N-Iodsuccinimid und 3-(12-Brom-1- tert.-butyl-dimethyl-silyl -2,3-dihydro-6-methyl-8α-ergolinyl)1,1-diethyl-harnstoff den 1,1-Diethyl-3-(2,3-dihydro-12-iod-6-methyl-8α-ergolinyl)-harnstoff

Mit Iod und 3-(13-Brom-1- tert.-butyl-dimethyl-silyl -2,3-dihydro-6-methyl-8α-ergolinyl)-1,1-diethyl-harnstoff den 1,1-Diethyl-3-(2,3-dihydro-13-iod-6-methyl-8α-ergolinyl)-harnstoff

Mit Iod und 3-(12-Brom-1- tert.-butyl-dimethyl-silyl -9,10-didehydro-6-methyl-8α-ergolinyl)-1,1-diethyl-harnstoff den 3-(9,10-Didehydro-12-iod-6-methyl-8α-ergolinyl)1,1-diethyl-harnstoff

Mit Iod und 3-(13-Brom-1- tert.-butyl-dimethyl-silyl -6-n-propyl-8α-ergolinyl)-1,1-diethyl-harnstoff den 1,1-Diethyl-3-(13-iod-6-methyl-8α-ergolinyl)-harnstoff

## Beispiel 2

### 1.1-Diethyl-3-(13-hydroxy-6-methyl-8α-ergolinyl)-harnstoff

419 mg 3-(13-Brom-6-methyl-8α-ergolinyl)-1,1-diethyl-harnstoff (1 mMol) löst man in 15 ml wasserfreiem, frisch destillierten THF unter Argon, kühlt auf - 20 °C und versetzt mit 5 ml einer 1,4 molaren Lösung von tert.-Butyllithium in Hexan. Man läßt 2 Stunden bei 0 °C rühren, kühlt dann auf - 70 °C ab und versetzt mit 0,5gNitrobenzol in 10 ml wasserfreiem, frisch destillierten THF. Nach 10 Minuten Rühren gibt man die Mischung auf Eis, macht mit 25 %iger Ammoniaklösung alkalisch und schüttelt mit Methylenchlorid aus. Der Rückstand wird durch Chromatographie an Kieselgel gereinigt und aus Essigester und Diisopropylester kristallisiert, Ausbeute 131 mg (37 % d. Th.),

$[\alpha]_D$ = +11 ° (0,5 % in Methanol).

Analog werden hergestellt:

Mit Nitrobenzol und 3-(12-Brom-9,10-didehydro-6-methyl-8α-ergolinyl)-1,1-diethyl-harnstoff der 1,1-Diethyl-3-(9,10-didehydro-12-hydroxy-6-methyl-8α-ergolinyl)-harnstoff, Ausbeute 41 %

Mit Methanthiosulfonsäure-S-methylester und 3-(13-Brom-6-methyl-8α-ergolinyl)-1,1-diethyl-harnstoff der 1,1-Diethyl-3-(6-methyl-13-methylthio-8α-ergolinyl)-harnstoff in 46 % Ausbeute.

$[\alpha]_D$ = - 13 ° (0,25 % in Chloroform)

Mit Methylisothiocyanat und 3-(13-Brom-6-methyl-8α-ergolinyl)-1,1-diethyl-harnstoff das 8α-(3,3-Diethylureido)-6-methyl-ergolin-13-thiocarbonsäure-methylamid in 42 % Ausbeute.

$[\alpha]_D$ = + 32 ° (0,5 % in Chloroform)

Mit Dimethylformamid und 3-(12-Brom-6-methyl-8α-ergolinyl)-1,1-diethyl-harnstoff der 1,1-Diethyl-3-(12-formyl-6-methyl-8α-ergolinyl)-harnstoff in 70 % Ausbeute.

$[\alpha]_D$ = + 20,6 ° (0,5 % in Chloroform).

Mit Dimethylformamid und 3-(12-Brom-2,3-dihydro-6-methyl-8α-ergolinyl)-1,1-diethylharnstoff der 1,1-Diethyl-3-(12-formyl-2,3-dihydro-6-methyl-8α-ergolinyl)-harnstoff in 65 % Ausbeute.

$[\alpha]_D$ = + 14 ° (0,5 % in Chloroform).

Mit Dimethylacetamid und 3-(12-Brom-6-methyl-8α-ergolinyl)-1,1-diethylharnstoff 3-(12-Acetyl-6-methyl-8α-ergolinyl)-1,1-diethylharnstoff in 23 % Ausbeute.

$[\alpha]_D$ = + 57,5 ° (0,5 % in Chloroform).

Mit Dimethylcarbonat und 3-(12-Brom-6-methyl-8α-ergolinyl)-1,1-diethylharnstoff der 8 α-(3,3-Diethylureido)-6-methyl-ergolin-12-carbonsäuremethylester in 47 % Ausbeute.

$[\alpha]_D$ = + 72 ° ( 0,5 % in Chloroform).

Mit Kohlendioxid und 3-(12-Brom-6-methyl-8α-ergolinyl)-1,1-diethylharnstoff die 8α-(3,3-Diethylureido)-6-methyl-ergolin-12-carbonsäurein 40 % Ausbeute.

Mit Trifluoressigsäureanhydrid und 3-(12-Brom-6-methyl-8α-ergolinyl)-1,1-diethylharnstoff der 1,1-Diethyl-3-(6-methyl-12-trifluoroacetyl-8α-ergolinyl)-harnstoff in 19 % Ausbeute.
$[\alpha]_D$ = + 6 ° (0,25 % in Chloroform).

Beispiel 3

1,1-Diethyl-3-(6-methyl-13-methylsulfinyl-8α-ergolinyl)-harnstoff

Man löst 210 mg 1,1-Diethyl-3-(6-methyl-13-methylthio-8α-ergolinyl)-harnstoff in 20 ml Acetonitril und gibt portionsweise Natriummetaperiodat, gelöst in 5 ml Wasser, zu. Man rührt 16 Stunden bei 50 °C, verteilt den Rückstand zwischen Methylenchlorid und Wasser, trocknet die organische Phase mit Natriumsulfat und dampft ein. Der Rückstand wird an Kieselgel chromatographiert.

Analog wird hergestellt: Mit Natriummetaperiodat und 1,1-Diethyl-3-(6-methyl-12-methylthio-8α-ergolinyl)-harnstoff der 1,1-Diethyl-3-(6-methyl-12-methylsulfinyl-8α-ergolinyl)-harnstoff, Ausbeute 48 % d.Th.
$[\alpha]_D$ = + 28,8 ° (0,5 % in Chloroform).

Beispiel 4

3-(13-Acetoxy-6-methyl-8α-ergolinyl)-1,1-diethyl-harnstoff

Man löst 100 mg 1,1-Diethyl-3-(13-hydroxy-6-methyl-8α-ergolinyl)-harnstoff in 1 ml Pyridin und 1 ml Essigsäureanhydrid bei Raumtemperatur auf. Nach einer Stunde gießt man auf Eis, extrahiert nach 15 Minuten Rühren mit Methylenchlorid, trocknet die organische Phase mit Natriumsulfat und dampft ein. Der Rückstand wird aus Essigester kristallisiert.

Beispiel 5

3-(12-Cyan-6-methyl-8α-ergolinyl)-1,1-diethyl-harnstoff

383 mg[8α-(3,3-Diethylureido)-6-methyl-ergolin-12-yl]-carbonsäureamid (1mMol) löst man in 25 ml Chloroform, gibt 3 ml Phosphoroxychlorid zu und rührt 16 Stunden bei 55 °C. Die Mischung wird auf Eis gegossen, 30 Minuten gewartet, dann mit 1n Kalilauge alkalisch gemacht und mit Methylenchlorid extrahiert. Die organische Phase wird mit Natriumsulfat getrocknet und eingedampft. Der Rückstand kristallisiert auf Essigester in 43 % Ausbeute.
$[\alpha]_D$ = + 16 ° (0,5 % in Chloroform).

Beispiel 6

1.1-Diethyl-3-(6-methyl-13-hydroxymethyl-8α-ergolinyl)-harnstoff

Man suspendiert 80 mg Lithiumaluminiumhydrid (2 mMol) in 5 ml wasserfreiem, frisch destillierten THF und gibt bei Raumtemperatur eine Lösung von 350 mg 1,1-Diethyl-3-(13-formyl-6-methyl-8α-ergolinyl)-harnstoff (1 mMol) gelöst in 10 ml wasserfreiem, frisch destillierten THF zu. Nach einer Stunde Rühren bei Raumtemperatur versetzt man unter Kühlung mit 5 ml 1n Salzsäure, gibt 5 ml 2n Weinsäurelösung zu und macht mit wässrigem Ammoniak alkalisch. Man extrahiert mit Essigester, trocknet die organische Phase und dampft sie ein. Nach Chromatographie an Kieselgel wird die Substanz aus Essigester kristallisiert.

Analog werden hergestellt:

Mit Lithiumaluminiumhydrid und 1,1-Diethyl-3-(12-formyl-6-methyl-8α-ergolinyl)-harnstoff der 1,1-Diethyl-3-(12-hydroxymethyl-6-methyl-8α-ergolinyl)-harnstoff in 51 % Ausbeute.
$[\alpha]_D$ = + 47° (0,5 % in Chloroform).

Mit Lithiumaluminiumhydrid und 1,1-Diethyl-3-(2,3-dihydro-12-formyl-6-methyl-8α-ergolinyl)-harnstoff der 1,1-Diethyl-3-(2,3-dihydro-12-hydroxymethyl-8α-ergolinyl)-harnstoff als Hydrogenfumarat in 33 % Ausbeute.
$[\alpha]_D$ = - 3,2 ° (0,5 % in Methanol).

Beispiel 7

1,1-Diethyl-3-(12-hydroxy-6-methyl-8α-ergolinyl)-harnstoff

Eine Lösung von 67 mg Diisopropylamin in 0,5ml absolutem Tetrahydrofuran wird unter Argonatmosphäre und Rühren bei 0 - 5 °C mit 0,42 mln-Butyllithium ( 15 %ig in Hexan) versetzt, die Mischung auf - 20 °C gekühlt, eine Lösung von 305 mg 3-(12-Brom-1-tert.-butyl-dimethyl-silyl-6-methyl-8α-ergolinyl)-1,1-diethylharnstoff in 2,5 ml absolutem Tetrahydrofuran zugetropft und 30 Minuten bei dieser Temperatur gerührt. Die Mischung wird auf - 70 °C gekühlt, 3 ml tert.-Butyllithium (2,3 molar in Pentan) tropfenweise zugegeben und 30 Minuten bei dieser Temperatur gerührt. Dann werden bei - 70 °C 0,62 ml Borsäuretrimethylester zugefügt, 15 Minuten bei dieser Temperatur und 2 Stunden bei Raumtemperatur gerührt. Die Mischung wird auf - 10 °C gekühlt, nacheinander mit 0,13 ml Eisessig, einer Mischung aus 0,34 ml 30 %igen Wasserstoffperoxid und 0,34 ml Wasser versetzt und 20 Minuten bei 0 °C gerührt.

Die Reaktionsmischung wird auf Eis gegeben, mit 25 %iger Ammoniaklösung alkalisch gestellt und mit Dichlormethan extrahiert. Die vereinigten organischen Phasen werden mit einer 10 %igen Ammoniumeisensulfatlösung gewaschen, mit Magnesiumsulfat getrocknet und eingeengt. Das erhaltene Produkt wird in 1 ml Trifluoressigsäure gelöst, 3 Stunden bei + 5 °C gerührt, die Reaktionsmischung auf Eis gegeben, mit 25 %iger Ammoniaklösung alkalisch gestellt und mit Dichlormethan extrahiert.

Die vereinigten organischen Phasen werden über Magnesiumsulfat getrocknet, eingeengt und das Rohprodukt an Kieselgel mit Dichlormethan/Methanol/25 %iger Ammoniaklösung im Verhältnis 98 : 2 : 0,1 chromatographiert. Man erhält so 50 mg 1,1-Diethyl-3-(12-hydroxy-6-methyl-8α-ergolinyl)-harnstoff.
$[\alpha]_D$ = + 18,4 ° (0,5 % in Chloroform).

Beispiel 8

1,1-Diethyl-3-(6-methyl-12-methylthio-8α-ergolinyl)-thioharnstoff

Man löst 5,79 g 1,1-Diethyl-3-(6-methyl-12-methylthio-8α-ergolinyl)-harnstoff (15 mMol) in einer Mischung von 4,13g frisch destilliertem Phosphoroxychlorid (45 mMol) und 50 ml wasserfreiem Methylenchlorid bei - 20 °C und läßt die Temperatur in 4 Stunden auf + 10 °C kommen. Über Nacht wird bei Raumtemperatur, dann noch 2 Stunden bei 40 °C gerührt und anschließnd das Lösungsmittel im Vakuum abdestilliert. Der Rückstand wird in 50 ml wasserfreiem Acetonitril gelöst, auf - 10 °C abgekühlt mit 7,2 g Kalium-ethylxanthogenat (45 mMol) versetzt und 20 Stunden bei Raumtemperatur gerührt. Das Lösungsmittel wird weitgehend abdestilliert, dann verteilt man zwischen Essigester und gesättigter Natriumcarbonatlösung, trocknet die organische Phase mit Natriumsulfat und dampft ein. Der Rückstand wird aus Essigester kristallisiert, Ausbeute 82 %.
$[\alpha]_D$= + 48 ° (0,5 % in Chloroform).

Analog werden die folgenden Thioharnstoffe aus den entsprechenden Harnstoffen dargestellt:
1,1-Diethyl-3-(6-methyl-13-methylthio-8α-ergolinyl)-thioharnstoff
8α-(3,3-Diethylthioureido)-6-methyl-ergolin-13-thiocarbonsäure-methylamid
1,1-Diethyl-3-(2,3-dihydro-6-methyl-13-methylthio-8α-ergolinyl)-thioharnstoff
3-(9,10-Didehydro-6-methyl-12-methylthio-8α-ergolinyl)-1,1-diethyl-thioharnstoff
1,1-Diethyl-3-(13-methylthio-6-n-propyl-8α-ergolinyl)-thioharnstoff

Beispiel 9

3-(12-Cyan-2,3-dihydro-6-methyl-8α-ergolinyl)-1,1-diethylharnstoff

Eine Suspension von 500 mg 1,1-Diethyl-3-(2,3-dihydro-12-formyl-6-methyl-8α-ergolinyl)-harnstoff und 460 mg Hydroxylamin-O-sulfonsäure in 5 ml Wasser wird 20 Stunden bei Raumtemperatur gerührt. Die Reaktionsmischung wird auf Eis gegeben, mit 25 %iger Ammoniaklösung alkalisch gestellt und mit Dichlormethan extrahiert. Die organische Phase wird getrocknet ($Na_2SO_4$), eingeengt, der Rückstand an Kieselgel mit Dichlormethan / Methanol = 95 / 5 als Elutionsmittel chromatographiert und aus Ethylacetat / Pentan kristallisiert. Ausbeute 26 % d.Th.
$[\alpha]_D$ = + 26 ° (0,5 % in Chloroform).

## Patentansprüche

**1.** 12- oder 13-substituierte Ergolinderivate der allgemeinen Formel I

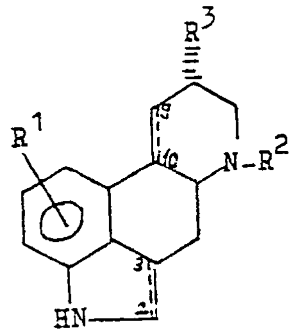

(I)

worin

$R^1$ eine OR'-Gruppe (R' = H, $C_{1-6}$-Alkyl, $C_{2-5}$-Alkanoyl), eine SH, $SR^5$ oder $SOR^5$-Gruppe ($R^5$ = $C_{1-6}$-Alkyl, Phenyl, Phenyl-$C_{1-2}$-Alkyl), eine

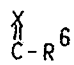 -Gruppe,

worin x = O oder S bedeutet und $R^6$ = H, $CF_3$, $C_{1-6}$-Alkyl, eine gegebenenfalls mit $C_{1-6}$-Alkyl substituierte Aminogruppe oder $OR^7$ bedeutet mit $R^7$ in der Bedeutung von Wasserstoff oder $C_{1-6}$-Alkyl,
eine $-CR^8R^9R^{10}$-Gruppe
mit $R^8$ = H, OH, O-$C_{2-5}$-Alkanoyl, O-$C_{1-6}$-Alkyl, $C_{1-6}$-Alkyl oder eine gegebenenfalls mit $C_{1-6}$-Alkyl substituierte Aminogruppe,
$R^9$ und $R^{10}$ gleich oder verschieden sind und jeweils H, $C_{1-6}$-Alkyl oder Phenyl bedeuten,
eine $SO_2CF_3$-, $Si(CH_3)_3$ oder CN-Gruppe, ein Cl- oder J-Atom bedeutet und

$R^2$ eine $C_{1-6}$-Alkylgruppe ist und

$R^3$ NH-CO-$NEt_2$ oder NH-CS-$NEt_2$ bedeutet und

$C_9...C_{10}$ und $C_2...C_3$ eine CC-Einfach- oder eine C=C Doppelbindung bedeuten und das Wasserstoffatom in 10-Stellung α-ständig ist, wenn $C_9...C_{10}$ eine CC-Einfachbindung ist und das Wasserstoffatom in 3-Stellung α- oder β-ständig ist, wenn $C_2...C_3$ eine CC-Einfachbindung ist, sowie deren Säureadditionssalze.

**2.** 1,1-Diethyl-3-(6-methyl-13-methylthio-8α-ergolinyl)-harnstoff

1,1-Diethyl-3-(6-methyl-12-methylthio-8α-ergolinyl)-harnstoff

1,1-Diethyl-3-(2,3-dihydro-6-methyl-12-methylthio-8α-ergolinyl)-harnstoff

3-(9,10-Didehydro-6-methyl-12-methylthio-8α-ergolinyl)-1,1-diethyl-harnstoff

1,1-Diethyl-3-(13-methylthio-6-n-propyl-8α-ergolinyl)-harnstoff

1,1-Diethyl-3-(2,3-dihydro-13-methylthio-6-n-propyl-8α-ergolinyl)-harnstoff

1,1-Diethyl-3-(12-ethylthio-6-methyl-8α-ergolinyl)-harnstoff

1,1-Diethyl-3-(6-methyl-13-phenylthio-8α-ergolinyl)harnstoff

1,1-Diethyl-3-(2,3-dihydro-12-hydroxy-6-methyl-8α-ergolinyl)-harnstoff

1,1-Diethyl-3-(2,3-dihydro-13-hydroxy-6-methyl-8α-ergolinyl)-harnstoff

3-(9,10-Didehydro-12-hydroxy-6-methyl-8α-ergolinyl)-1,1-diethylharnstoff

1,1-Diethyl-3-(13-hydroxy-6-n-propyl-8α-ergolinyl)-harnstoff

8α-(3,3-Diethylureido)-6-methyl-ergolin-12-carbonsäureamid

8α-(3,3-Diethyl-ureido)-6-methyl-ergolin-13-thiocarbonsäuremethylamid

1,1-Diethyl-3-(13-formyl-6-methyl-8α-ergolinyl)-harnstoff

1,1-Diethyl-3-(13-dimethyl-aminomethyl-6-methyl-8α-ergolinyl)-harnstoff

1,1-Diethyl-3-(6,13-dimethyl-8α-ergolinyl)-harnstoff

1,1-Diethyl-3-(6-methyl-13-iso-propyl-8α-ergolinyl)-harnstoff

3-(13-Acetyl-6-methyl-8α-ergolinyl)-1,1-diethyl-harnstoff

3-(13-Chlor-6-methyl-8α-ergolinyl)-1,1-diethyl-harnstoff

3-(13-Chlor-2,3-dihydro-6-methyl-8α-ergolinyl)-1,1-diethyl-harnstoff

1,1-Diethyl-3-(13-iod-6-methyl-8α-ergolinyl)-harnstoff

1,1-Diethyl-3-(2,3-dihydro-12-iod-6-methyl-8α-ergolinyl)-harnstoff

1,1-Diethyl-3-(2,3-dihydro-13-iod-6-methyl-8α-ergolinyl)-harnstoff

1,1-Diethyl-3-(13-hydroxy-6-methyl-8α-ergolinyl)-harnstoff

1,1-Diethyl-3-(9,10-didehydro-12-hydroxy-6-methyl-8α-ergolinyl)-harnstoff

1, 1-Diethyl-3-(6-methyl-13-methylthio-8α-ergolinyl)-harnstoff

8α-(3,3-Diethylureido)-6-methyl-ergolinyl-13-thiocarbonsäure-methylamid

3-(13-Acetoxy-6-methyl-8α-ergolinyl)-1,1-diethyl-harnstoff

3-(12-Cyan-6-methyl-8α-ergolinyl)-1,1-diethyl-harnstoff

1,1-Diethyl-3-(6-methyl-13-hydroxymethyl-8α-ergolinyl)-harnstoff

1,1-Diethyl-3-(12-hydroxy-6-methyl-8α-ergolinyl)-harnstoff

1,1-Diethyl-3-(6-methyl-12-methylthio-8α-ergolinyl)-thioharnstoff

1,1-Diethyl-3-(6-methyl-13-methylthio-8α-ergolinyl)-thioharnstoff

8α-(3,3-Diethylthioureido)-6-methyl-ergolin-13-thiocarbonsäure-methylamid

1,1-Diethyl-3-(2,3-dihydro-6-methyl-13-methylthio-8α-ergolinyl)-thioharnstoff

3-(9,10-Didehydro-6-methyl-12-methylthio-8α-ergolinyl)-1,1-diethyl-thioharnstoff

1,1-Diethyl-3-(13-methylthio-6-n-propyl-8α-ergolinyl)-thioharnstoff

nach Anspruch 1.

**3.** Verfahren zur Herstellung der Verbindungen der allgemeinen Formel I, dadurch gekennzeichnet, daß man ein 12- oder 13-Br-Ergolinderivat der allgemeinen Formel II

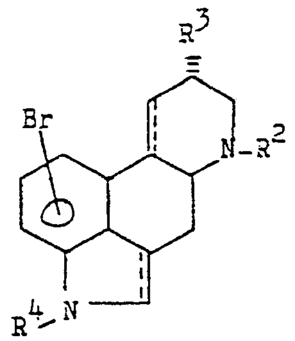

(II)

worin $R^2$, $R^3$ und C---C die obige Bedeutung haben und $R^4$ Wasserstoff oder eine Schutzgruppe ist, mit einem Lithium-Organyl umsetzt und das so erhaltene 12- oder 13-Li-Ergolinderivat der allgemeinen Formel III

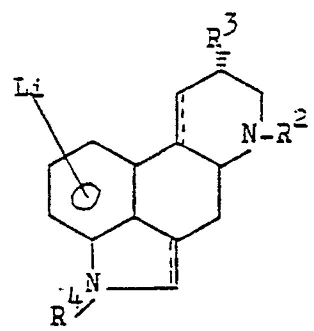

(III)

worin $R^4$, $R^2$, $R^3$ und C---C die obige Bedeutung haben, mit einem elektrophilen Reagenz umsetzt und gewünschtenfalls anschließend eine OH-Gruppe alkyliert oder acyliert, eine Carboxylfunktion verestert, eine Aldehydfunktion reduziert, ein Carbonsäureamid in ein Nitril überführt, ein Schwefelatom oxidiert oder einen Harnstoff in einen Thioharnstoff umwandelt und gegebenenfalls mit einer Säure in das physiologisch verträgliche Säureadditionssalz überführt.

**4.** Verwendung der Verbindungen gemäß Anspruch 1 und 2 als Arzneimittel

**5.** Arzneimittel auf Basis der Verbindungen nach Anspruch 1 und 2.

## Claims

**1.** 12- or 13-substituted ergoline derivatives of the general formula I

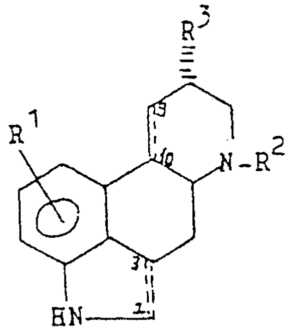

(I)

wherein
$R^1$ is an OR' group (R' = H, $C_{1-6}$-alkyl, $C_{2-5}$-alkanoyl), an SH, $SR^5$ or $SOR^5$ group ($R^5$ = $C_{1-6}$-alkyl, phenyl, phenyl-$C_{1-2}$-alkyl), or a

$$\overset{X}{\overset{\|}{C}}-R^6 \text{ group,}$$

wherein X = O or S and $R^6$ = H, $CF_3$, $C_{1-6}$-alkyl, an amino group optionally substituted by $C_{1-6}$-alkyl, or = $OR^7$ in which $R^7$ is hydrogen or $C_{1-6}$-alkyl,
a $-CR^8R^9R^{10}$ group
in which $R^8$ = H, OH, O-$C_{2-5}$-alkanoyl, O-$C_{1-6}$-alkyl, $C_{1-6}$-alkyl or an amino group optionally substituted by $C_{1-6}$-alkyl,
$R^9$ and $R^{10}$ are identical or different and each is H, $C_{1-6}$-alkyl or phenyl,
an $SO_2CF_3$, $Si(CH_3)_3$ or CN group, a Cl or I atom and

$R^2$ is a $C_{1-6}$-alkyl group and

$R^3$ is NH-CO-$NEt_2$ or NH-CS-$NEt_2$ and

each of $C_9...C_{10}$ and $C_2...C_3$ is a CC single bond or a C=C double bond and the hydrogen atom in the 10-position is in the $\alpha$-configuration if $C_9...C_{10}$ is a CC single bond, and the hydrogen atom in the 3-position is in the $\alpha$- or $\beta$-configuration if $C_2...C_3$ is a CC single bond, as well as the acid addition salts thereof.

**2.** 1,1-diethyl-3-(6-methyl-13-methylthio-8$\alpha$-ergolinyl)-urea

1,1-diethyl-3-(6-methyl-12-methylthio-8$\alpha$-ergolinyl)urea

1,1-diethyl-3-(2,3-dihydro-6-methyl-12-methylthio-8$\alpha$-ergolinyl)urea

3-(9,10-didehydro-6-methyl-12-methylthio-8$\alpha$-ergolinyl)-1,1-diethylurea

1,1-diethyl-3-(13-methylthio-6-n-propyl-8$\alpha$-ergolinyl)urea

1,1-diethyl-3-(2,3-dihydro-13-methylthio-6-n-propyl-8$\alpha$-ergolinyl)urea

1,1-diethyl-3-(12-ethylthio-6-methyl-8$\alpha$-ergolinyl)urea

1,1-diethyl-3-(6-methyl-13-phenylthio-8$\alpha$-ergolinyl)urea

1,1-diethyl-3-(2,3-dihydro-12-hydroxy-6-methyl-8$\alpha$-ergolinyl)urea

1,1-diethyl-3-(2,3-dihydro-13-hydroxy-6-methyl-8$\alpha$-ergolinyl)urea

3-(9,10-didehydro-12-hydroxy-6-methyl-8$\alpha$-ergolinyl)-1,1-diethylurea

1,1-diethyl-3-(13-hydroxy-6-n-propyl-8$\alpha$-ergolinyl)urea

8$\alpha$-(3,3-diethylureido)-6-methylergoline-12-carboxylic acid amide

8$\alpha$-(3,3-diethylureido)-6-methylergoline-13-thiocarboxylic acid methylamide

1,1-diethyl-3-(13-formyl-6-methyl-8$\alpha$-ergolinyl)urea

1,1-diethyl-3-(13-dimethylaminomethyl-6-methyl-8$\alpha$-ergolinyl)urea

1,1-diethyl-3-(6,13-dimethyl-8$\alpha$-ergolinyl)urea

1,1-diethyl-3-(6-methyl-13-isopropyl-8α-ergolinyl)urea

3-(13-acetyl-6-methyl-8α-ergolinyl)-1,1-diethylurea

3-(13-chloro-6-methyl-8α-ergolinyl)-1,1-diethylurea

3-(13-chloro-2,3-dihydro-6-methyl-8α-ergolinyl)-1,1-diethylurea

1,1-diethyl-3-(13-iodo-6-methyl-8α-ergolinyl)urea

1,1-diethyl-3-(2,3-dihydro-12-iodo-6-methyl-8α-ergolinyl)urea

1,1-diethyl-3-(2,3-dihydro-13-iodo-6-methyl-8α-ergolinyl)urea

1,1-diethyl-3-(13-hydroxy-6-methyl-8α-ergolinyl)urea

1,1-diethyl-3-(9,10-didehydro-12-hydroxy-6-methyl-8α-ergolinyl)urea

1,1-diethyl-3-(6-methyl-13-methylthio-8α-ergolinyl)urea

8α-(3,3-diethylureido)-6-methylergolinyl-13-thiocarboxylic acid methylamide

3-(13-acetoxy-6-methyl-8α-ergolinyl)-1,1-diethylurea

3-(12-cyano-6-methyl-8α-ergolinyl)-1,1-diethylurea

1,1-diethyl-3-(6-methyl-13-hydroxymethyl-8α-ergolinyl)-urea

1,1-diethyl-3-(12-hydroxy-6-methyl-8α-ergolinyl)urea

1,1-diethyl-3-(6-methyl-12-methylthio-8α-ergolinyl)thiourea

1,1-diethyl-3-(6-methyl-13-methylthio-8α-ergolinyl)thiourea

8α-(3,3-diethylthioureido)-6-methylergoline-13-thiocarboxylicacid methylamide

1,1-diethyl-3-(2,3-dihydro-6-methyl-13-methylthio-8α-ergolinyl)thiourea

3-(9,10-didehydro-6-methyl-12-methylthio-8α-ergolinyl)-1,1-diethylthiourea

1,1-diethyl-3-(13-methylthio-6-n-propyl-8α-ergolinyl)-thiourea

according to claim 1.

3. A process for the preparation of the compounds of the general formula I, characterised in that a 12- or 13-Br-ergoline derivative of the general formula II

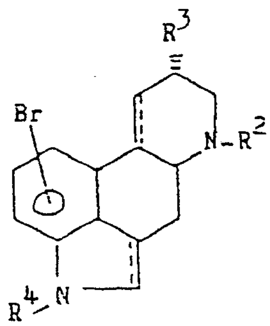

(II)

wherein $R^2$, $R^3$ and C...C have the meanings given above and $R^4$ is hydrogen or a protecting group, is reacted with a lithium organyl and the resulting 12- or 13-Li-ergoline derivative of the general formula III

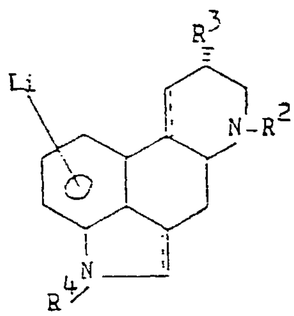

(III)

wherein $R^4$, $R^2$, $R^3$ and C...C have the meanings given above, is reacted with an electrophilic reagent and, if desired, subsequently an OH group is alkylated or acylated, a carboxy function is esterified, an aldehyde function is reduced, a carboxylic acid amide is converted into a nitrile, a sulphur atom is oxidised or a urea is converted into a thiourea, and optionally converted with an acid into a physiologically compatible acid addition salt.

**4.** Use of the compounds according to claims 1 and 2 as medicaments.

**5.** Medicament based on the compounds according to claims 1 and 2.

**Revendications**

**1.** Ergolines substituées en position 12 ou 13 qui répondent à la formule générale I :

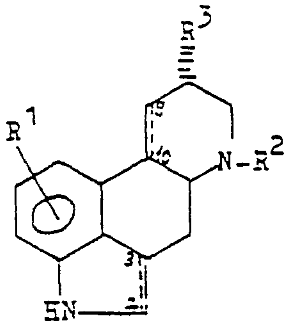

(I)

dans laquelle
$R^1$ représente
- un radical OR' dans lequel R' représente H, un alkyle en $C_1$-$C_6$ ou un alcanoyle en $C_2$-$C_5$,

- un radical -SH,
- un radical $-SR^5$ ou $-SOR^5$ dont le symbole $R^5$ représente un alkyle en $C_1$-$C_6$, un phényle ou un phénylalkyle dont l'alkyle contient un ou deux atomes de carbone,
- un radical

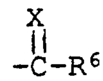

dans lequel X représente O ou S et $R^6$ représente H, $-CF_3$, un alkyle en $C_1$-$C_6$, un radical amino éventuellement porteur d'un alkyle en $C_1$-$C_6$ ou un radical $-OR^7$ dont le symbole $R^7$ représente l'hydrogène ou un alkyle en $C_1$-$C_6$,
- un radical $-CR^8R^9R^{10}$ dans lequel $R^8$ représente H, -OH, un alcanoyloxy en $C_2$-$C_5$, un alcoxy en $C_1$-$C_6$, un alkyle en $C_1$-$C_6$ ou un radical amino éventuellement porteur d'un alkyle en $C_1$-$C_6$, et $R^9$ et $R^{10}$ représentent chacun, indépendamment l'un de l'autre, H, un alkyle en $C_1$-$C_6$ ou un phényle,
- un radical $-SO_2CF_3$, $-Si(CH_3)_3$ ou -CN, ou
- un atome de chlore ou d'iode,

$R^2$ représente un radical alkyle en $C_1$-$C_6$,
$R^3$ représente un radical -NH-CO-NEt$_2$ ou -NH-CS-NEt$_2$ et
$C_9$---$C_{10}$ et $C_2$---$C_3$ représentent chacun une liaison simple CC ou une liaison double C=C, l'atome d'hydrogène à la position 10 ayant la configuration $\alpha$ lorsque $C_9$---$C_{10}$ représente une liaison simple CC, et l'atome d'hydrogène à la position 3 ayant la configuration $\alpha$ ou $\beta$ lorsque $C_2$---$C_3$ représente une liaison simple CC,
et sels d'addition formés par ces composés avec des acides.

**2.** Composés selon la revendication 1, en l'espèce :

la diéthyl-1,1 (méthyl-6 méthylthio-13 ergolinyl-8$\alpha$)-3 urée,

la diéthyl-1,1 (méthyl-6 méthylthio-12 ergolinyl-8$\alpha$)-3 urée,

la diéthyl-1,1 (dihydro-2,3 méthyl-6 méthylthio-12 ergolinyl-8$\alpha$)-3 urée,

la (didéhydro-9,10 méthyl-6 méthylthio-12 ergolinyl-8$\alpha$)-3 diéthyl-1,1 urée,

la diéthyl-1,1 (méthylthio-13 n-propyl-6 ergolinyl-8$\alpha$)-3 urée,

la diéthyl-1,1 (dihydro-2,3 méthylthio-13 n-propyl-6 ergolinyl-8$\alpha$)-3 urée,

la diéthyl-1,1 (éthylthio-12 méthyl-6 ergolinyl-8$\alpha$)-3 urée,

la diéthyl-1,1 (méthyl-6 phénylthio-13 ergolinyl-8$\alpha$)-3 urée,

la diéthyl-1,1 (dihydro-2,3 hydroxy-12 méthyl-6 ergolinyl-8$\alpha$)-3 urée,

la diéthyl-1,1 (dihydro-2,3 hydroxy-13 méthyl-6 ergolinyl-8$\alpha$)-3 urée,

la (didéhydro-9,10 hydroxy-12 méthyl-6 ergolinyl-8$\alpha$)-3 diéthyl- 1,1 urée,

la diéthyl-1,1 (hydroxy-13 n-propyl-6 ergolinyl-8$\alpha$)-3 urée,

le (diéthyl-3,3 uréido)-8$\alpha$ méthyl-6 ergoline-carboxamide-12,

le (diéthyl-3,3 uréido)-8$\alpha$ méthyl-6 ergoline-(N-méthyl-thiocarboxamide)-13,

la diéthyl-1,1 (formyl-13 méthyl-6 ergolinyl-8$\alpha$)-3 urée,

la diéthyl-1,1 (diméthylaminométhyl-13 méthyl-6 ergolinyl-8α)-3 urée,

la diéthyl-1,1 (diméthyl-6,13 ergolinyl-8α)-3 urée,

la diéthyl-1,1 (méthyl-6 isopropyl-13 ergolinyl-8α)-3 urée,

l'(acétyl-13 méthyl-6 ergolinyl-8α)-3 diéthyl-1,1 urée,

la (chloro-13 méthyl-6 ergolinyl-8α)-3 diéthyl-1,1 urée,

la (chloro-13 dihydro-2,3 méthyl-6 ergolinyl-8α)-3 diéthyl-1,1 urée,

la diéthyl-1,1 (iodo-13 méthyl-6 ergolinyl-8α)-3 urée,

la diéthyl-1,1 (dihydro-2,3 iodo-12 méthyl-6 ergolinyl-8α)-3urée,

la diéthyl-1,1 (dihydro-2,3 iodo-13 méthyl-6 ergolinyl-8α)-3urée,

la diéthyl-1,1 (hydroxy-13 méthyl-6 ergolinyl-8α)-3 urée,

la diéthyl-1,1 (didéhydro-9,10 hydroxy-12 méthyl-6 ergolinyl-8α)-3 urée,

la diéthyl-1,1 (méthyl-6 méthylthio-13 ergolinyl-8α)-3 urée,

le méthylamide de l'acide (diéthyl-3,3 uréido)-8α méthyl-6 ergoline-thiocarboxylique-13,

l'(acétoxy-13 méthyl-6 ergolinyl-8α)-3 diéthyl-1,1 urée,

la (cyano-12 méthyl-6 ergolinyl-8α)-3 diéthyl-1,1 urée,

la diéthyl-1,1 (méthyl-6 hydroxyméthyl-13 ergolinyl-8α)-3 urée,

la diéthyl-1,1 (hydroxy-12 méthyl-6 ergolinyl-8α)-3 urée,

la diéthyl-1,1 (méthyl-6 méthylthio-12 ergolinyl-8α)-3 thio-urée,

la diéthyl-1,1 (méthyl-6 méthylthio-13 ergolinyl-8α)-3 thio-urée,

le méthylamide de l'acide (diéthyl-3,3 thio-uréido)-8α méthyl-6ergoline-thiocarboxylique-13,

la diéthyl-1,1 (dihydro-2,3 méthyl-6 méthylthio-13 ergolinyl-8α)-3 thio-urée,

la (didéhydro-9,10 méthyl-6 méthylthio-12 ergolinyl-8α)-3 diéthyl-1,1 thio-urée, et

la diéthyl-1,1 (méthylthio-13 n-propyl-6 ergolinyl-8α)-3 thio-urée.

3. Procédé pour préparer les composés de formule générale I, procédé caractérisé en ce qu'on fait réagir une bromo-12 ou -13 ergoline répondant à la formule générale II :

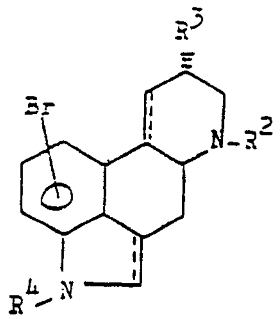

.(II)

dans laquelle $R^2$, $R^3$ et C---C ont les significations qui leur ont été données ci-dessus et $R^4$ représente l'hydrogène ou un radical protecteur,
avec un organyl-lithium, on fait réagir la lithium-12 ou -13 ergoline ainsi obtenue, qui répond à la formule générale III :

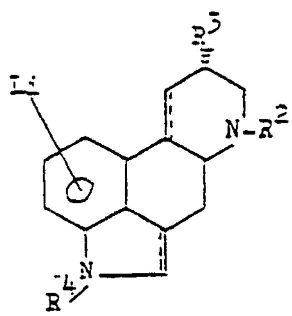

(III)

dans laquelle $R^4$, $R^2$, $R^3$ et C---C ont les significations qui leur ont été données ci-dessus,
avec un réactif électrophile, puis, si on le désire, on alkyle ou acyle un radical -OH, on estérifie une fonction carboxy, on réduit une fonction aldéhyde, on transforme un carboxamide en un nitrile, on oxyde un atome de soufre et/ou on transforme une urée en une thio-urée, et éventuellement on transforme le composé obtenu, par réaction avec un acide, en un sel d'addition acceptable du point de vue physiologique.

**4.** Application des composés selon l'une des revendications 1 et 2 comme médicaments.

**5.** Médicaments à base de composés selon l'une des revendications 1 et 2.